# EUROPEAN PATENT APPLICATION

(11) **EP 0 759 305 A2**
(43) Date of publication of application: **26.02.1997**
(21) Application number: 96305480.4
(22) Date of filing: 25.07.1996
(51) Int. Cl.: A61L 17/00

(54) **Braided polyester suture**

(30) Priority: 26.07.1995 US 1516; 02.02.1996 US 595541
(71) Applicant: ETHICON INC., Somerville New Jersey 08876 (US)
(72) Inventor: D'Aversa, Margaret M., Whitehouse Station, NJ 08889 (US); Hunt, Geoffrey T., Edingburgh, EH14 1AJ (GB); Washington, Robert, Mt. Airy, GA 30563 (US)
(74) Representative: Mercer, Christopher Paul

(57) **Abstract**

The present invention provides an improved braided polyester suture made from polyester filaments having a weight average molecular weight of greater than 35,000, a tenacity of from about 7 to about 8.5 grams /denier, a percent elongation to break of less than 30 percent and a boiling point water shrinkage of from about 0.5 to 3.0 percent. The inventive suture has reduced roughness and improved tensile strength.

## Description

### Field of the Invention

This application relates to braided sutures and more specifically braided polyester sutures.

### Background of the Invention

Braided polyester sutures have been available for many years. They are often used in cardiovascular surgery where it is desirable to have a strong nonabsorbable suture to permanently repair tissue. However, surgeons are always seeking better sutures that are easier to handle.

One area of suture handling that surgeons are concerned about is the ease with which a secure knot can be tied in a suture. The surgeon would generally like to be able to use a standard surgeon's knot and tighten the suture down into a secure knot with a minium effort. If a suture has a rough surface it may give the surgeon an uneven feel when a knot is tied in the suture (which is referred to as "chattering"). Chattering is caused by surface friction between the suture surfaces. To compensate for the surface friction surgeons exert additional force while tying knot which may result in suture breakage. With a suture that has a rough surface it is difficult for the surgeon to determine whether enough force has been exert to provide a secure knot.

Commonly, to reduce suture roughness, sutures are coated with a lubricous coating. However, if too much coating is applied the suture may become too slippery to easily handle and the knots tied in a suture may come undone.

We have discovered an improved braided polyester suture that has reduced suture roughness, as well as, good knot strength and improved tensile strength.

### Summary of the Invention

We have discovered that an improved braided polyester suture can be made from a polyester yarn made from polyester filaments having a weight average molecular weight of greater than 35,000, a tenacity of greater than 6 grams/denier, an elongation to break of less than 35%, a boiling water shrinkage in the range of from about 0.5 to about 2.0 percent, and the yarn made from these filaments have in the range of from 4 to about 15 turn per inch said yarn being braided into a suture.

### Brief Description of the Drawings

Fig. 1 is a diagrammatic representation of an Instron Tester and shows two braided suture strands in position for testing.

Fig. 2 is an enlarged perspective view of the single throw knot illustration in Fig. 1.

Fig. 3 is a representative illustration of the output from measurement of the roughness of a suture.

### Detailed Description of the Invention

We have discovered that an improved braided polyester suture can be produced by using polyethylene terephthalate filaments (hereinafter polyester) having certain properties. The individual polyester filaments should have a weight average molecular weight of greater than 35,000 preferably greater than 40,000 and most preferably in the range of from about 42,000 to about 45,000. The filaments should have a tenacity of greater than 6 grams/denier, preferably greater than 7 grams/denier and most preferably in the range of from about 7 grams/denier to about 8.5 grams/denier. The filaments should have a percent elongation to break of less than 35%, preferably less than 25%, and most preferably less than 20%. The filaments should have a boiling water shrinkage in the range of from about 0.5 to about 2.0 percent based on the original length of the filament. The filament should have a hot air shrinkage at 350°C of in the range of from about 3 to about 5 percent (compared to the original length). Preferred are commercially available filaments that may be purchased from Hoechst Celanese under the trademark Trevira™ High Tenacity type 712 and 787 polyester yarns.

The polyester yarns should be made up of filaments having a denier in the range of from about 0.2 to about 6 and preferably a denier from about 1.2 to about 3.4 and more preferably a denier of from about 1.4 to about 3.1. The polyester filaments are extruded in bundles (yarns) having a denier in the range of from about 20 to about 500 denier and preferably 20 to 350 denier. Preferably the yarns will be twisted to have in the range of from about 4 to about 15 turns per inch (TPI) and most preferably 8 TPI.

The twisted yarns produced above can be braided into sutures using conventional braid constructions having a sheath and optionally a core. The construction of the braid will preferably be within the parameters provided in Table I below, but could also utilize the braid constructions described in U.S. patent No. 5,019,093 (which is hereby incorporated by reference).

**Table I**

| **Braid Constructions** | | | | | | |
|---|---|---|---|---|---|---|
| *Suture Size* | *Total Denier in the range of about* | *Denier of Filaments in the range of about* | *Number Sheath Yarns in the range of about* | *Carriers in the range of about* | *Core Denier in the range of about* | *Picks Count in the range of about* |
| *2* | *1900-2,600* | *3.4-3.0* | *40-110* | *12-24* | *350-775* | *35-50* |
| *1* | *1,500-1,900* | *3.3-3.0* | *40-110* | *12-24* | *300-500* | *35-50* |
| *0* | *1200 - 1500* | *2.5-1.8* | *40-110* | *12-24* | *175-375* | *35-50* |
| *2/0* | *800-1,200* | *2.0-2.4* | *40-110* | *8-24* | *75-200* | *35-50* |
| *3/0* | *500-800* | *1.6-2.0* | *40-110* | *8-24* | *30-60* | *35-50* |
| *4/0* | *300-500* | *1.6-2.0* | *40-110* | *8-24* | *--* | *35-50* |
| *5/0* | *200-300* | *1.3-1.7* | *40-110* | *8-24* | *--* | *35-50* |

Alternatively, a spiral braid pattern may be used such as was described in U.S. Patent No. 4,959,069, filed September 25, 1990 and U.S. Patent No. 5,059,213 filed October 22, 1991 (both of which are hereby incorporated by reference). Most preferably to provide the best combination of suture properties the sutures will be braided using the suture constructions provided in Table II below.

**Table II**

| **Preferred Suture Construction** | | | | |
|---|---|---|---|---|
| *Suture Size* | *Sheath Yarns* | *Carriers* | *Core Denier* | *Picks* |
| *2* | *90-150* | *12-18* | *380-500* | *40-50* |
| *1* | *55-90* | *14-18* | *310-360* | *40-50* |
| *0* | *60-80* | *14-18* | *180-250* | *40-50* |
| *2/0* | *45-65* | *14-18* | *80-140* | *40-50* |
| *3/0* | *35-55* | *10-14* | *40-50* | *40-50* |
| *4/0* | *35-55* | *6-10* | *--* | *40-50* |

After the braid has been made the braided suture may be stretched in the presence of heat. The braided suture may preferably be hot stretched at a temperature in the range of from about 160°C to about 250°C and stretched to increase its length from about 9 to 28 percent of its initial.

The suture may be coated with an absorbable or nonabsorbable coating material. Currently it is preferred to coat the suture within the range of from about 0.1 to about 10 weight percent polybutilate coating (calculated by comparing the precoating weight with the weight of the suture after coating and removal of any excess solvent). Coatings can be applied using any conventional method. For applying polybutilate it is preferred to use a suspension drop coating system.

The sutures produced as described above have improved properties such as reduced suture roughness, and knot slide force and improved tensile strength (as compared to commercially available sutures, as described in Example 4). For example size 2/0 sutures prepared according to the present invention have an average suture roughnesses measured as described in Example 3 of less than 60 grams and preferably will have an average suture roughness of in the range of from about 57 to about 43 grams and/or suture knot slide values of less than 1 lb, preferably less than 0.5 lbs and most preferably in the range of from about 0.5 to about 0.25 lbs.

The stretched braided suture may have one or more needles attached and sterilized using conventional means (i.e. ethylene oxide or irradiation).

### Example 1

55 denier Trevira™ type 787 polyester yarn (Hoechst Celanese Corporation) was wound onto braider bobbins using a Hacoba bobbin winding machine. The bobbins were transferred to a New England Butt braider. The braider was configured to provide a braid construction with 16 carriers, with a core denier of 110 and a pick count of 50. The braided suture was then wound under tension onto a pressure dyeing tube. The suture was placed in the tube dyeing unit and scoured with detergent to remove any lubricants that might have been applied to aid in braiding. The braided suture was then stretched under tension to meet the desired finished diameter and elongation requirements. The suture was next coated with a polybutilate coating solution to give the suture improved handling characteristics. Finally needles were attached, the suture was packaged and sterilized.

### Example 2

The absolute knot tensile strength of the inventive suture prepared as described in Example 1 was compared to the roughness of two commercially available sutures. These values are not normalized by suture diameter (Table V provides intrinsic knot tensile strength normalized by diamter).

The knot tensile strength of the sutures were determined by the following procedure. A square throw was introduced into each suture by passing one end of the suture through the loop and tightening it. Each suture was placed taut in the INSTRON Tensile Tester with the knot approximately midway between the clamps, tested at a 12.7 cm gauge length and 30.5 cm/min crosshead speed.

**TABLE III**

| **Knot Tensile Strength of 2/0 Polyester Suture** | | | |
|---|---|---|---|
| *NAME* | *SAMPLE NO.* | *KNOT TENSILE* | |
| *Inventive Suture* | | *lbs.* | *S.D.* |
| | *1* | *7.42* | *0.50* |
| *"* | *2* | *7.37* | *0.57* |
| *"* | *3* | *7.61* | *0.59* |
| *TEVDEK II* | *4* | *7.31* | *0.38* |
| *"* | *5* | *7.75* | *0.46* |
| *"* | *6* | *8.08* | *0.52* |
| *"* | *7* | *7.51* | *0.43* |
| | *8* | *7.87* | *0.60* |
| *TICRON* | *9* | *6.33* | *0.61* |
| *"* | *10* | *6.61* | *0.45* |
| *"* | *11* | *7.04* | *0.45* |
| *"* | *12* | *7.00* | *0.55* |

### Example 3

The roughness of an inventive suture prepared as described in Example 1 was compared to the roughness of three commercially available sutures.

The roughness of a suture is important because it indicates the drag or chatter that a suture will have as it is drawn through tissue. Sutures with higher roughness values are more difficult to draw through tissue and do more damage to the tissue as the suture is pulled through the tissue. Roughness will also play a role in sliding knots down to tie sutures together, again higher values are undesirable.

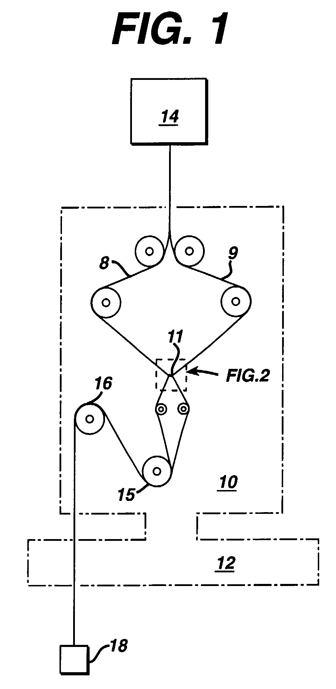

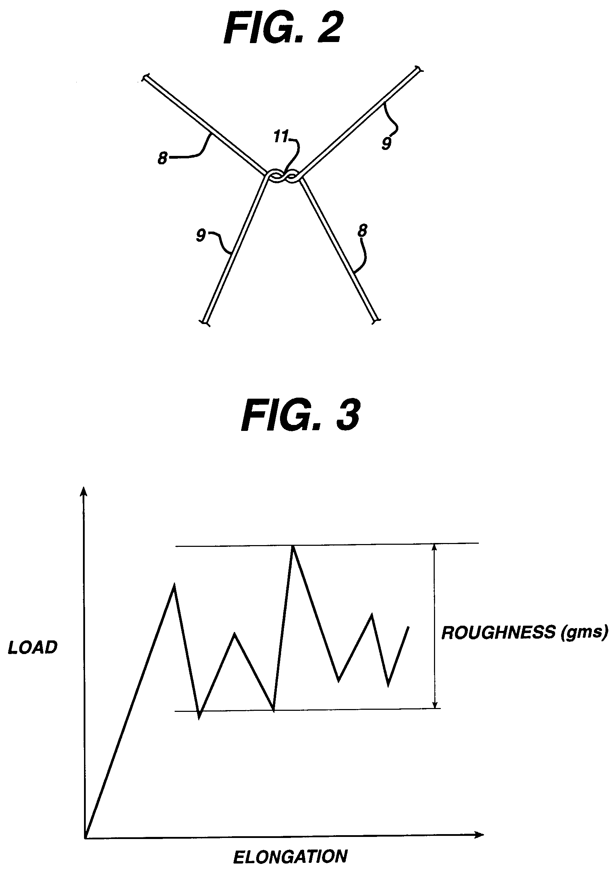

In the roughness test, two strands **8** and **9** of the same suture are attached at the end of the load cell **14** (100 kg) of an Instron tester (Constant Rate of Extension Tensile Testing Machine). The sutures were threaded through the pulleys **15** and **16** and attached to the crosshead **12** of the Instron tester (as shown in Figure 1 and 2). The other end of the suture strands were attached together to a weight **18** (2.5 lbs for size 2/0 suture) which provides tension as would be in a subjective tie-down evaluation. As the Instron crosshead moves down, the "knot" **11** slides along the suture for a definite length at the same speed as the crosshead. The total downward force exerted by the upper end of the suture on the transducer is equal to the tension weight plus the force required to slide the knot. The amplitude of the load elongation curve (as shown in Figure 3) gives the estimated "roughness" of the suture which is a measure of suture-to-suture friction.

**Table IV**

| **Roughness and Knot Slide of 2/0 Polyester Suture** | | | | | |
|---|---|---|---|---|---|
| Suture Roughness | Sample No. | Suture Roughness | | Knot Slide | |
| | | gms | s.d. | lbs. | s.d. |
| Inventive Suture | 1 | 57.34 | 17.33 | 0.35 | 0.35 |
| | 2 | 43.56 | 9.56 | 0.25 | 0.05 |
| | 3 | 44.54 | 14.45 | 0.26 | 0.06 |
| TVDEKII™ | 4 | 67.68 | 19.37 | 2.13 | 0.49 |
| | 5 | 80.47 | 24.40 | 2.35 | 0.59 |
| | 6 | -- | -- | 1.99 | 0.50 |
| | 7 | -- | -- | 1.24 | 0.32 |
| | 8 | 67.68 | 19.37 | 2.21 | 0.36 |
| TICRON™ | 9 | 113.38 | 37.94 | 1.76 | 0.66 |
| | 10 | -- | -- | 2.37 | 0.69 |
| | 11 | -- | -- | 1.87 | 0.54 |
| | 12 | 94.85 | 23.66 | 2.23 | 0.39 |
| | 13 | 117.76 | 34.11 | 2.29 | 0.41 |
| SURGIDAC™ | 14 | 135.15 | 38.16 | 3.75 | 0.31 |
| | 15 | 207.42 | 186.61 | 3.48 | 0.36 |
| | 16 | 322.13 | 155.82 | 2.61 | 1.01 |
| | 17 | -- | -- | 2.88 | 0.54 |
| | 18 | -- | -- | 1.55 | 0.81 |

### Example 4

The properties of the inventive suture were compared to several commercially available sutures. The results of these tests are described below in Table V.

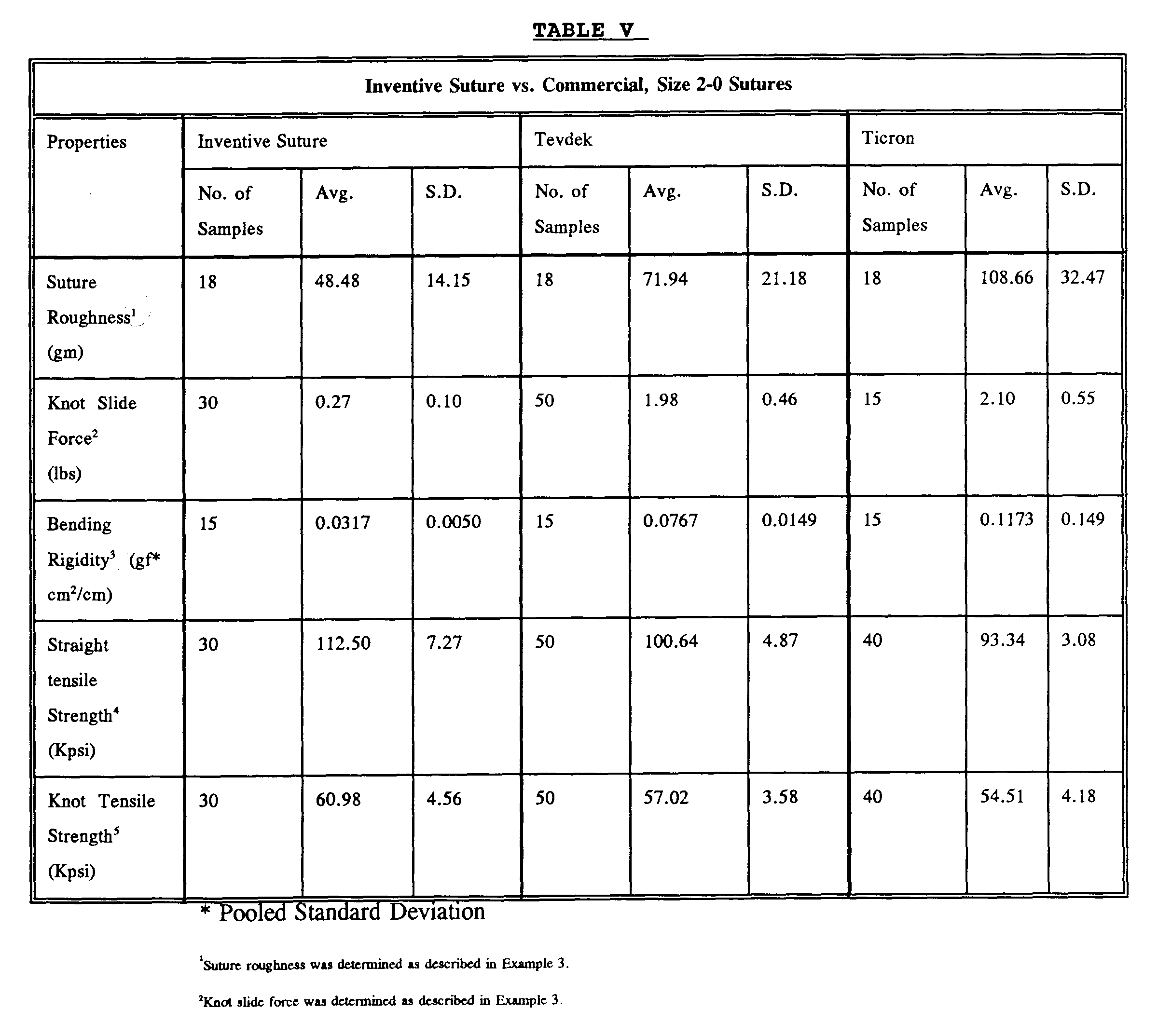

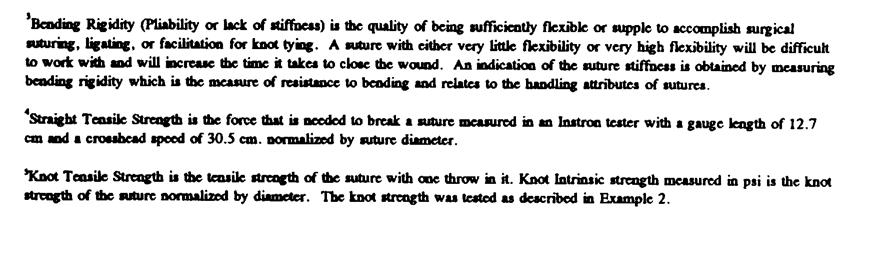

## Claims

1. A braided polyester suture constructed from yarn filaments having a weight average molecular weight of greater than 35,000, a tenacity of from about 7 to about 8.5 grams/denier, a percent elongation to break of less than 30 percent and a boiling point water shrinkage of from about 0.5 to 3.0 percent.

2. The braided polyester suture of claim 1 wherein the braided suture has the following construction:
| Suture Size | Total Denier in the range of about | Denier of Filaments in the range of about | Number Sheath Yarns in the range of about | Carriers in the range of about | Core Denier in the range of about | Picks Count in the range of about |
|---|---|---|---|---|---|---|
| 2 | 1,900-2,600 | 3.4-3.0 | 40-110 | 12-24 | 350-775 | 35-50 |
| 1 | 1,500-1,900 | 3.3-3.0 | 40-110 | 12-24 | 300-500 | 35-50 |
| 0 | 1,200-1,900 | 2.5-1.8 | 40-110 | 12-24 | 175-375 | 35-50 |
| 2/0 | 800-1,200 | 2.0-2.4 | 40-110 | 8-24 | 75-200 | 35-50 |
| 3/0 | 500-800 | 1.6-2.0 | 40-110 | 8-24 | 30-60 | 35-50 |
| 4/0 | 300-500 | 1.6-2.0 | 40-110 | 8-24 | -- | 35-50 |
| 5/0 | 200-300 | 1.3-1.7 | 40-110 | 8-24 | -- | 35-50 |
wherein the yarn used to make the suture is twisted in the range of from about 4 to about 15 turns per inch

3. The braided polyester suture of claim 1 or claim 2 wherein the yarn has a weight average molecular weight greater than 40,000,preferably in the range of from about 42,000 to about 45,000.

4. The braided polyester suture of any one of claims 1 to 3 wherein the braided suture has the following construction:
| *Suture Size* | *Sheath Yarns* | *Carriers* | *Core Denier* | *Picks* |
|---|---|---|---|---|
| *2* | *90-150* | *12-18* | *380-500* | *40-50* |
| *1* | *55-90* | *14-18* | *310-360* | *40-50* |
| *0* | *60-80* | *14-18* | *180-250* | *40-50* |
| *2/0* | *45-65* | *14-18* | *80-140* | *40-50* |
| *3/0* | *35-55* | *10-14* | *40-50* | *40-50* |
| *4/0* | *35-55* | *6-10* | *--* | *40-50* |

5. A size 2/0 braided polyester suture with an average suture roughness of less than 60 grams, preferably in the range of from about 57 to about 43 grams.

6. A size 2/0 braided polyester suture with an average knot slide force of less than 1 lb, preferably less than 0.5 lbs, most preferably in the range of from about 0.5 lbs to about 0.25 lbs.

7. The size 2/0 braided polyester suture of claim 5 or claim 6 wherein the size 2/0 braided polyester suture has knot security at five throws using the Herman's Loop Test.
